# EUROPEAN PATENT APPLICATION

(11) **EP 4 775 604 A1**
(43) Date of publication of application: **15.07.2026**
(21) Application number: 24862693.9
(22) Date of filing: 29.08.2024
(51) Int. Cl.: C08F 30/08, C07F 7/08, C07F 7/18, G02C 7/04

(54) **POLYMERIZABLE SILICONE MONOMER, METHOD FOR PRODUCING SAME, CURABLE COMPOSITION, POLYMER THEREOF, AND OPHTHALMIC DEVICE USING SAME**

(30) Priority: 07.09.2023 JP 2023145534
(71) Applicant: SHIN-ETSU CHEMICAL CO., LTD., Tokyo 100-0005 (JP)
(72) Inventor: KUDO, Muneo, Annaka-shi, Gunma 379-0224 (JP); UEHARA, Hitoshi, Annaka-shi, Gunma 379-0224 (JP); URATA, Minoru, Suita-shi, Osaka 564-0034 (JP)
(74) Representative: Ipsilon
(86) International application number: PCT/JP2024/030972
(87) International publication number: WO 2025/053041

(57) **Abstract**

[Object] To provide a polymerizable silicone monomer that yields a polymer having excellent transparency, surface hydrophilicity, durability, and stain resistance, and a method for preparing the polymerizable silicone monomer. Another object of the present invention is to provide a curable composition containing the polymerizable silicone monomer, a polymer obtained by polymerizing the composition, and an ophthalmic device comprising the polymer.

[Solution] A polymerizable silicone monomer represented by the following formula (1): 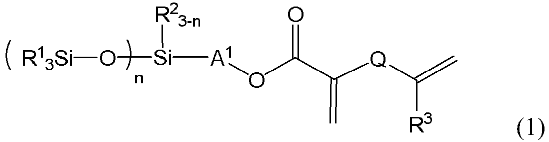 wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms, R³ is a hydrogen atom or a methyl group, A¹ is a single bond or a divalent hydrocarbon group having 1 to 20 carbon atoms, Q is a divalent group selected from the group consisting of -A²-O-A³-and -O-A⁴-O-, A² and A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, provided that A² and A³ are not both single bonds, A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and n is an integer of 1 to 3.

## Description

### TECHNICAL FIELD

The present invention relates to a polymerizable silicone monomer having an alkenyl ether moiety and an acryloyl group within the same molecule, a method for preparing the monomer, a curable composition comprising the monomer, a polymer obtained by polymerizing the composition, and an ophthalmic device comprising the polymer.

### BACKGROUND ART

Polymerizable silicone monomers are used as resin modifiers of (meth)acrylic resins, and are also copolymerized with other hydrophilic monomers for use in ophthalmic devices and the like. However, polymers obtained from conventional polymerizable silicone monomers have the problems of poor transparency, water absorption, toughness, and oxygen permeability.

Patent Literatures 1 and 2 describe a polymerizable compound having an allyl ether moiety and an acryloyl group within the same molecule, and a composition comprising the polymerizable compound, and state that the composition yields a polymer having excellent transparency, water absorbency, and toughness. The polymerizable compound has a property of undergoing cyclization polymerization via a radical polymerization mechanism. Polymers obtained from the composition containing the polymerizable compound exhibit superior transparency, water absorbency, and toughness as compared with polymers containing conventional polymerizable compounds.

### PRIOR LITERATURES

### PATENT LITERATURES

Patent Literature 1: Japanese Patent Application Laid-Open No. 2011-074068
Patent Literature 2: Japanese Patent Application Laid-Open No. 2011-137123

Known silicone monomers having polymerizable groups include silicone monomers having an acryloyl group or a methacryloyl group; however, there are few examples of silicone monomers having different α-substituent groups. In particular, there have been no reports of a polymerizable silicone monomer that has an alkenyl ether moiety and an acryloyl group within the same molecule and exhibits cyclization polymerization.

### SUMMARY OF THE INVENTION

### PROBLEM TO BE SOLVED BY THE INVENTION

The present invention has been made in view of the aforementioned circumstances, and one of the objects of the present invention is to provide a polymerizable silicone monomer that yields a polymer having excellent transparency, surface hydrophilicity, durability, and stain resistance, and a method for preparing the polymerizable silicone monomer. Another object of the present invention is to provide a curable composition containing the polymerizable silicone monomer, a polymer obtained by polymerizing the composition, and an ophthalmic device comprising the polymer.

### MEANS FOR SOLVING THE PROBLEMS

As a result of intensive studies conducted to achieve the aforementioned object, the present inventors synthesized a polymerizable silicone monomer having an alkenyl ether moiety and an acryloyl group within the same molecule, and found that a curable composition containing the polymerizable silicone monomer yields a polymer having excellent transparency, surface hydrophilicity, durability, and stain resistance, thereby accomplishing the present invention.

That is, the present invention provides
[1] a polymerizable silicone monomer represented by the following formula (1): 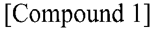 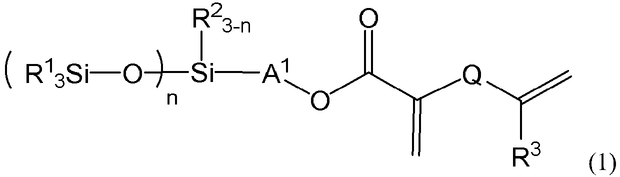 wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms, R³ is a hydrogen atom or a methyl group, A¹ is a single bond or a divalent hydrocarbon group having 1 to 20 carbon atoms, Q is a divalent group selected from the group consisting of -A²-O-A³-and -O-A⁴-O-, A² and A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, provided that A² and A³ are not both single bonds, A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and n is an integer of 1 to 3.

Furthermore, the present invention provides the polymerizable silicone monomer, further comprising at least one configuration among the following items [2] to [5].
[2] The polymerizable silicone monomer according to the item [1] represented by the following formula (a) or (b): 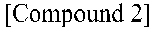 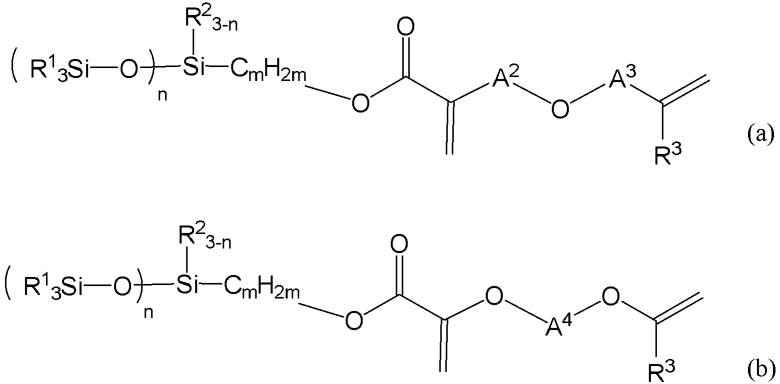 wherein R¹, R², R³, A², A³, A⁴ and n are as defined above, and m is an integer of 1 to 10.
[3] The polymerizable silicone monomer according to the item [1] or [2], wherein A², A³ and A⁴ are a group selected from the following groups. 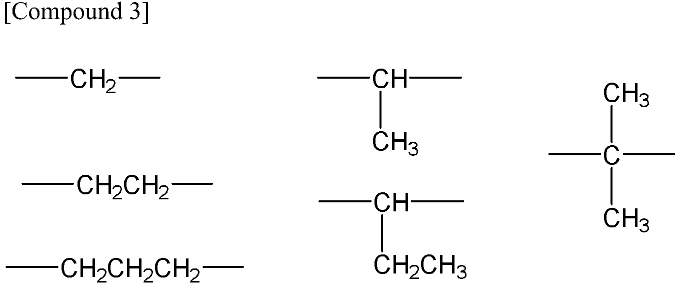
[4] The polymerizable silicone monomer according to any one of the items [1] to [3], wherein either A² or A³ is a single bond, and the other of A² and A³ is a group selected from the following groups: 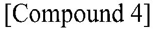 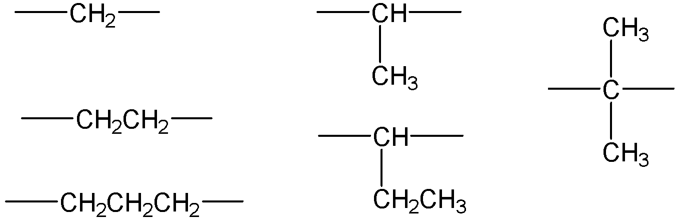
[5] The polymerizable silicone monomer according to any one of the items [1] to [4], wherein A², A³, and A⁴ are a methylene group.

Furthermore, the present invention provides
a method for preparing a polymerizable silicone monomer represented by the following formula (1): 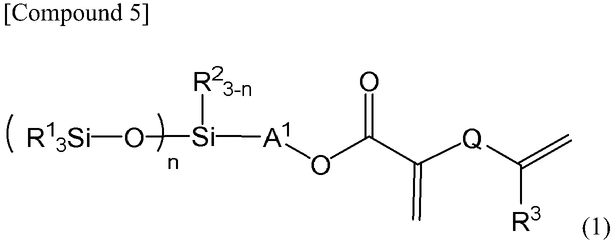 wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms, R³ is a hydrogen atom or a methyl group, A¹ is a single bond or a divalent hydrocarbon group having 1 to 20 carbon atoms, Q is a divalent group selected from the group consisting of -A²-O-A³-and -O-A⁴-O-, A² and A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, provided that A² and A³ are not both single bonds, A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and n is an integer of 1 to 3,
wherein the method comprises a step of reacting a halogen group-containing organosilicon compound represented by the following formula (2): 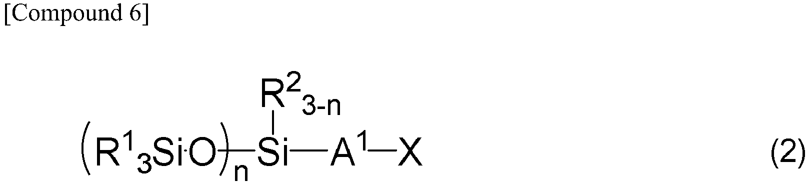 with a carboxylic acid metal salt compound represented by the following formula (3): 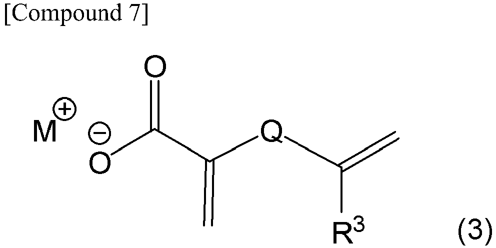
wherein R¹, R², R³, A¹, Q, and n are as defined above, X is a chlorine atom, a bromine atom, or an iodine atom, and M is an alkali metal ion,
to obtain the polymerizable silicone monomer represented by said formula (1).

Furthermore, the present invention provides the preparation method, further comprising at least one configuration among the following items [7] to [11].
[7] The preparation method according to the item [6], wherein the polymerizable silicone monomer represented by formula (1) is represented by said formula (a) or (b).
[8] The preparation method according to the item [6] or [7], wherein A², A³, and A ⁴ are groups selected from the following groups: 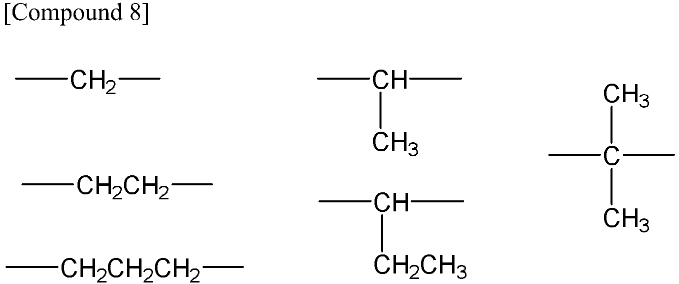
[9] The preparation method according to the item [6] or [7], wherein either A² or A³ is a single bond, and the other of A² and A³ is a group selected from the following groups: 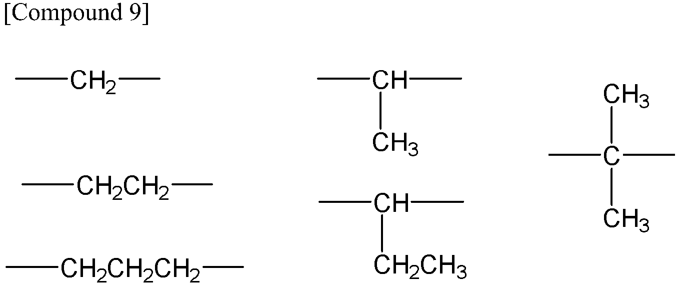
[10] The preparation method according to any one of the items [6] to [9], wherein A², A³ and A⁴ are a methylene group.
[11] The preparation method according to any one of items [6] to [10], wherein a molar ratio of the halogen group-containing organosilicon compound represented by formula (2) to the carboxylic acid metal salt compound represented by formula (3) is from 1.0:0.3 to 1.0:3.0.

Furthermore, the present invention provides [12] a method for preparing a polymerizable silicone monomer represented by the following formula (1): 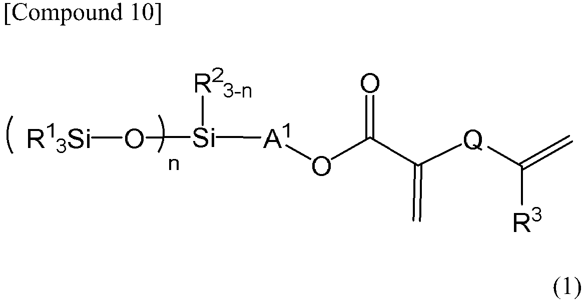 wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms, R³ is a hydrogen atom or a methyl group, A¹ is a single bond or a divalent hydrocarbon group having 1 to 20 carbon atoms, Q is a divalent group selected from the group consisting of -A²-O-A³-and -O-A⁴-O-, A² and A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, provided that A² and A³ are not both single bonds, A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and n is an integer of 1 to 3,
wherein the method comprises a step of subjecting an organosilicon compound represented by the following formula (4): 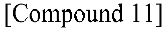 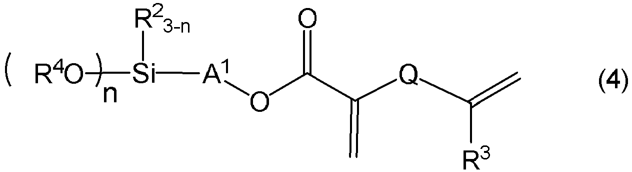 to a hydrolytic condensation reaction in the presence of an acid with a hydrolyzable organosilicon compound represented by the following formula (5) and/or a disiloxane compound represented by the following formula (6):
[Compound 12]

**V-SiR¹₃** (5)

**R¹₃SiOSiR¹₃** (6)

wherein R¹, R², R³, A¹, Q and n are as defined above, R⁴ is an alkyl group having 1 to 4 carbon atoms, and Y is a chlorine atom, a bromine atom, or an iodine atom,
to obtain the polymerizable silicone monomer represented by said formula (1).

Furthermore, the present invention provides the preparation method, further comprising at least one configuration among the following items [13] to [19].
[13] The preparation method according to the item [12], wherein the polymerizable silicone monomer represented by formula (1) is represented by said formula (a) or (b).
[14] The preparation method according to the item [12] or [13], wherein A², A³, and A⁴ are a group selected the following groups: 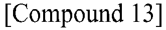 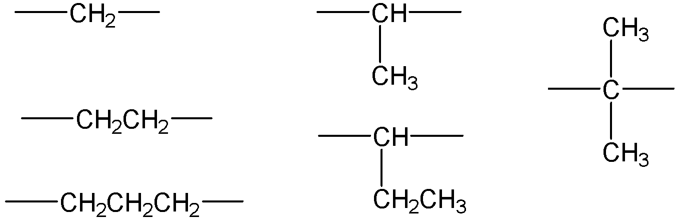
[15] The preparation method according to the item [12] or [13], wherein either A² or A³ is a single bond, and the other of A² and A³ is a group selected from the following groups: 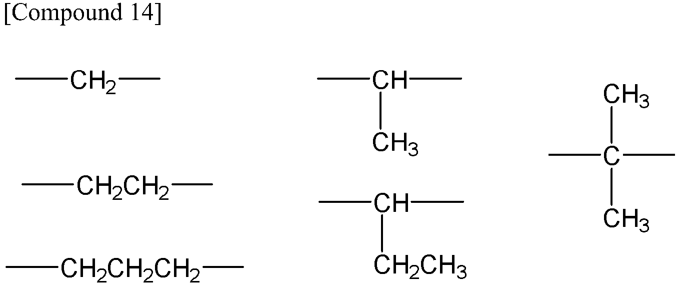
[16] The preparation method according to any one of the items [12] to [15], wherein A², A³, and A ⁴ are a methylene group.
[17] The preparation method according to any one of items [12] to [16], wherein the organosilicon compound represented by formula (4) is reacted with the hydrolyzable organosilicon compound represented by formula (5) at a blending ratio such that a molar ratio of the hydrolyzable organosilicon compound represented by formula (5) to the number of moles of OR⁴ groups contained in the organosilicon compound represented by formula (4) is from 1:1 to 1:10.
[18] The preparation method according to any one of the items [12] to [16], wherein the organosilicon compound represented by formula (4) is reacted with a disiloxane compound represented by the following formula (6) at a blending ratio such that a molar ratio of R¹₃Si groups contained in the disiloxane compound represented by formula (6) to the number of moles of OR⁴ groups contained in the organosilicon compound represented by formula (4) is from 1:1 to 1:10.
[19] The preparation method according to any one of the items [12] to [18], wherein an amount of the acid is 0.1 to 50 mol, per mol of the organosilicon compound represented by formula (4).

Furthermore, the present invention provides a curable composition comprising the polymerizable silicone monomer and another polymerizable monomer having a group that polymerizes with the polymerizable silicone monomer, a polymer obtained by polymerizing the curable composition, and an ophthalmic device comprising the polymer.

That is, there are provided:
[20] a curable composition comprising the polymerizable silicone monomer according to any one of the items [1] to [5] and another polymerizable monomer having a group that polymerizes with the polymerizable silicone monomer;
[21] the curable composition according to the item [14], wherein the curable composition comprises 10 to 80% by mass of the polymerizable silicone monomer according to any one of the items [1] to [5];
[22] a polymer obtained by polymerizing the curable composition according to the item [20] or [21]; and
[23] an ophthalmic device comprising the polymer according to the item [22].

There is further provided an ophthalmic device having at least one configuration among the following items [24] to [28].
[24] An ophthalmic device, comprising a polymer of a monomer mixture comprising the polymerizable silicone monomer according to any one of the items [1] to
[5] and another polymerizable monomer having a group that polymerizes with the polymerizable silicone monomer.
[25] The ophthalmic device according to the item [24], wherein a content ratio of the polymerizable silicone monomer represented by formula (1) is 10 to 80 parts by mass, relative to 100 parts by mass of the monomer mixture.
[26] The ophthalmic device according to the item [24] or [25], wherein the other polymerizable monomer is at least one selected from the group consisting of an acrylic monomer, an acrylic acid derivative, crotonic acid, cinnamic acid, vinyl benzoate, and a silicone monomer having a polymerizable group.
[27] The ophthalmic device according to any one of the items [24] to [26], wherein the monomer mixture further comprises at least one additive selected from the group consisting of a polymerization initiator, an antioxidant, a colorant, a lubricant, an internal wetting agent, and a reinforcing agent.
[28] The ophthalmic device according to any one of the items [24] to [27], wherein the ophthalmic device is at least one selected from the group consisting of a contact lens, an intraocular lens, and an artificial cornea.

A polymer obtained by polymerizing a curable composition containing a polymerizable silicone monomer of the present invention exhibits excellent transparency, surface hydrophilicity, durability, and stain resistance, and is therefore suitably used in ophthalmic devices and the like.

### MODE FOR CARRYING OUT THE INVENTION

The present invention will be explained in further detail below.

### Polymerizable silicone monomer

A polymerizable silicone monomer of the present invention is represented by the following formula (1).

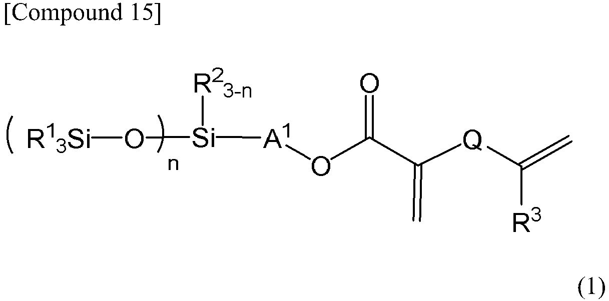

R¹ is, independently of each other, a monovalent hydrocarbon group having 1 to 4 carbon atoms. Examples of the monovalent hydrocarbon group include linear or branched alkyl groups such as methyl, ethyl, n-propyl, isopropyl, n-butyl, isobutyl, and *tert-*butyl groups. Among them, a methyl group or an ethyl group is preferred.

R² is, independently of each other, an alkyl group having 1 to 10 carbon atoms, preferably 1 to 8 carbon atoms, more preferably 1 to 6 carbon atoms, or an aryl group having 6 to 10 carbon atoms. The alkyl group may be any of linear, branched, or cyclic. Examples of the alkyl group include methyl, ethyl, n-propyl, isopropyl, n-butyl, isobutyl, *tert*-butyl*,* n-pentyl, neopentyl, n-hexyl, n-octyl, and cyclohexyl groups. Examples of the aryl group include phenyl, tolyl, xylyl, and naphthyl groups. Among them, R² is preferably an alkyl group and a phenyl group having 1 to 6 carbon atoms, more preferably a methyl group, an ethyl group, and a phenyl group.

R³ is a hydrogen atom or a methyl group.

A¹ is a single bond or a divalent hydrocarbon group having 1 to 20 carbon atoms. The divalent hydrocarbon group may be any of linear, branched-chain, and cyclic. Examples of the divalent hydrocarbon group include an alkylene group having 1 to 20 carbon atoms, preferably 1 to 10 carbon atoms, more preferably 1 to 6 carbon atoms; a cycloalkylene group having 3 to 20 carbon atoms; an alkenylene group having 2 to 10 carbon atoms; an arylene group having 6 to 10 carbon atoms; and an aralkylene group having 7 to 10 carbon atoms. More specifically, examples of the divalent hydrocarbon group include alkylene groups such as methylene, ethylene, propylene, trimethylene, tetramethylene, pentamethylene, hexamethylene, heptamethylene, and octamethylene groups; cycloalkylene groups such as a cyclohexylene group; alkenylene groups such as vinylene and propenylene groups; arylene groups such as phenylene, tolylene, xylylene, and naphthylene groups; and aralkylene groups such as benzylene, phenylethylene, and phenylpropylene groups.

"n" is an integer of 1 to 3, preferably 2 or 3.

Q is a divalent group selected from the group consisting of -A²-O-A³- and -O-A⁴-O-. A² and A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and provided that A² and A³ are not both single bonds. A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms.

Examples of the optionally branched divalent hydrocarbon group having 1 to 3 carbon atoms include alkylidene groups having 1 to 3 carbon atoms such as methylene, ethylidene, propylidene, and isopropylidene groups. A methylene group is preferred.

The polymerizable silicone monomer of the present invention is preferably represented by the following formula: 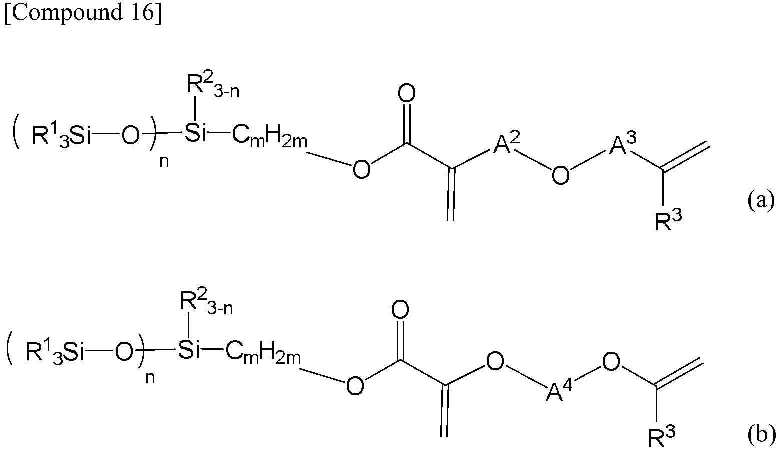 wherein R¹, R², R³, A², A³, A⁴ and n are as defined above, and m is an integer of 1 to 10.

Preferably, A², A³ and A⁴ are a group selected from the following structures.

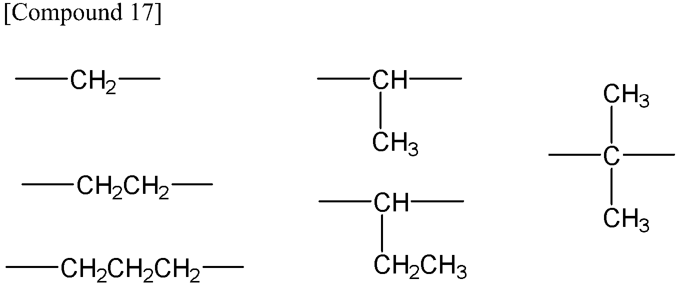

In another preferred embodiment, either A² or A³ is a single bond, and the other of A² or A³ is a group selected from the following formula.

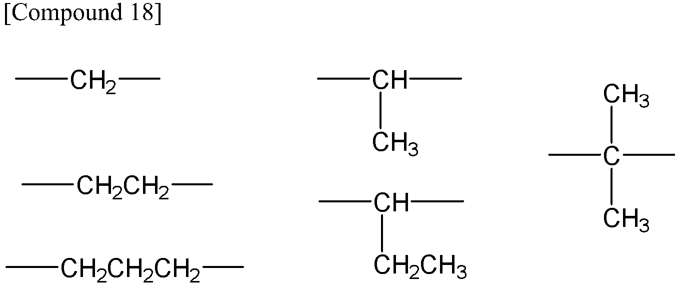

A², A³, and A⁴ are preferably a methylene group.

"m" is an integer of 1 to 10, preferably an integer of 2 to 10, more preferably an integer of 2 to 6.

The polymerizable silicone monomer of the present invention is more preferably represented by the following formula (8): 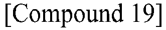 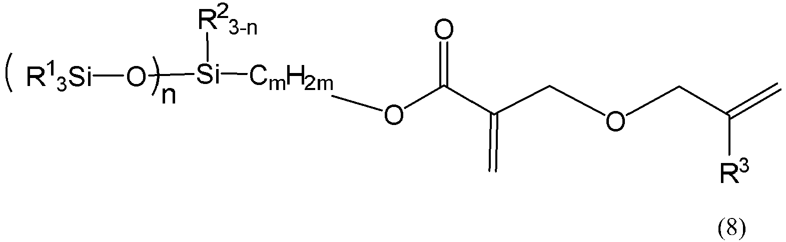 wherein R¹, R², m, and n are as defined above.

Examples of the polymerizable silicone monomer include compounds represented by the following formulas (9) to (11): 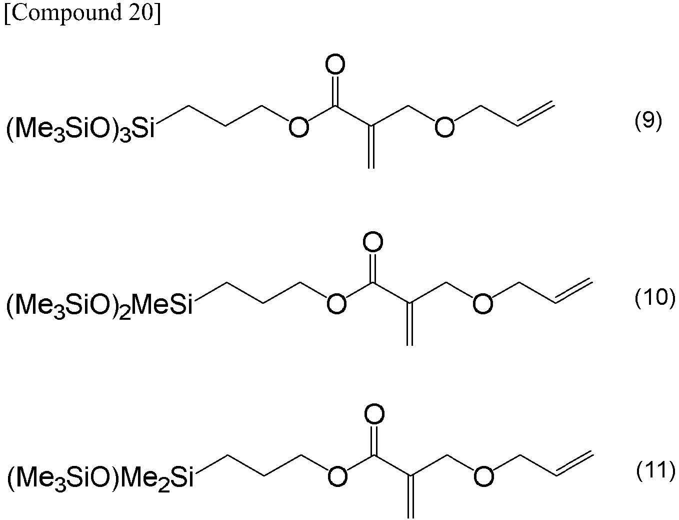 wherein Me represents a methyl group.

### Production Method 1

For example, the polymerizable silicone monomer of the present invention may be obtained by reacting a halogen group-containing organosilicon compound represented by the following formula (2) with a carboxylic acid metal salt compound represented by the following formula (3): 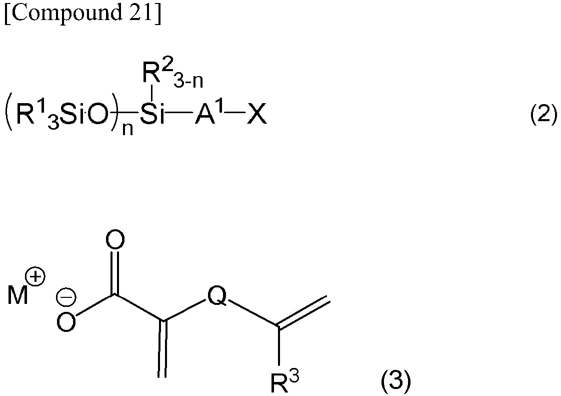 wherein R¹, R², R³, Q and n are as defined above, X is a chlorine atom, a bromine atom, or an iodine atom, and M is an alkali metal ion.

Examples of the alkali metal, M, include sodium and potassium.

Examples of the halogen group-containing organosilicon compound represented by formula (2) include 3-chloropropyl tris(trimethylsiloxy)silane, 3-chloropropylmethyl bis(trimethylsiloxy)silane, and 3-chloropropyldimethyl(trimethylsiloxy)silane.

More specifically, the carboxylic acid metal salt compound represented by formula (3) is represented by the following structure.

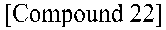

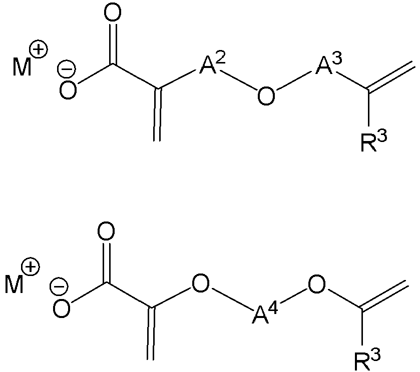

A², A³, and A⁴ are as defined above, preferably a group selected from the following groups. Furthermore, in another preferred embodiment, either A² or A³ is a single bond, and the other of A² and A³ is a group selected from the following groups:

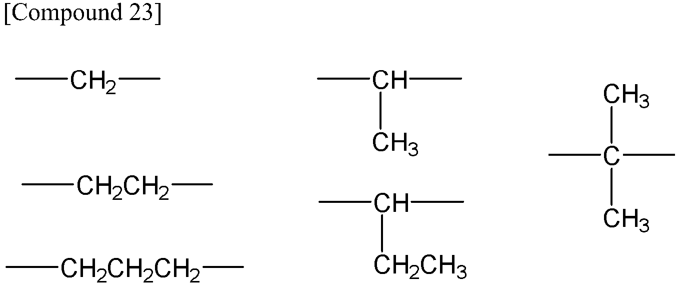

Most preferably A², A³, and A ⁴ are a methylene group.

Examples of the carboxylic acid metal salt compound represented by formula (3) include 2-(allyloxymethyl)sodium acrylate and 2-(allyloxymethyl)potassium acrylate.

It is preferred that the halogen group-containing organosilicon compound represented by formula (2) be reacted with the carboxylic acid metal salt compound represented by formula (3) at a molar ratio of halogen group-containing organosilicon compound to carboxylic acid metal salt compound of 1.0:0.3 to 1.0:3.0, particularly from 1:0.8 to 1:1.2.

In preparing the polymerizable silicone monomer of the present invention, a phase-transfer catalyst or another catalyst may be used, if necessary. Examples of the phase-transfer catalyst include quaternary phosphonium salts and quaternary ammonium salts.

Examples of quaternary phosphonium salts include tetraethylphosphonium chloride, tetraethylphosphonium bromide, tetraethylphosphonium iodide, tetrabutylphosphonium bromide, triphenylbenzylphosphonium bromide, and tetraphenylphosphonium bromide.

Examples of quaternary ammonium salts include tetramethylammonium hydroxide, tetraethylammonium hydroxide, trimethylbenzylammonium hydroxide, tetramethylammonium bromide, tetraethylammonium bromide, tetrabutylammonium bromide, trimethylbenzylammonium bromide, triethylbenzylammonium bromide, trimethylphenylammonium bromide, triethylbenzylammonium chloride, tetramethylammonium chloride, trioctylmethylammonium chloride, tributylbenzylammonium chloride, trimethylbenzylammonium chloride, N-laurylpyridinium chloride, *N*-benzylpicolinium chloride, *N*-lauryl 4-picolinium chloride, *N*-laurylpicolinium chloride, tricaprylmethylammonium chloride, tetramethylammonium iodide, tetra-n-butylammonium iodide, and tetrabutylammonium hydrogen sulfate.

When a catalyst is used, the amount of the catalyst used is not particularly limited, but in view of the reactivity and ease of preparation, the amount of the catalyst is preferably 0.1 to 10.0 parts by mass, more preferably 1.0 to 5.0 parts by mass, particularly preferably 2.0 to 4.0 parts by mass, relative to 100 parts by mass of the halogen group-containing organosilicon compound represented by formula (2).

In preparing the polymerizable silicone monomer of the present invention, a solvent may be used, if necessary, and the solvent is not particularly limited as long as it is nonreactive with the halogen group-containing organosilicon compound and the metal salt compound used as raw materials.

Examples of the solvent include aliphatic hydrocarbon solvents such as pentane, hexane, heptane, and decane; ether solvents such as diethyl ether and tetrahydrofuran; and aromatic hydrocarbon solvents such as benzene, toluene, and xylene. These solvents may be used alone or in combination of two or more thereof.

The reaction temperature is not particularly limited, and the reaction may be carried out under heating from 0 °C; however, a temperature of 0 to 200 °C is preferred. In order to obtain a moderate reaction rate, it is preferred to carry out the reaction under heating, and from this viewpoint, the reaction temperature is more preferably 40 to 110 °C, and even more preferably 40 to 90 °C.

Furthermore, the reaction time is not particularly limited, and typically is about 1 to 30 hours, preferably 1 to 20 hours, more preferably 1 to 10 hours.

An alkali metal halide generated during production of the polymerizable silicone monomer of the present invention can be separated from the target polymerizable silicone monomer by filtration or other known methods. The polymerizable silicone monomer of the present invention can be further purified by distillation under reduced pressure, liquid column chromatography, or other known methods.

### Production Method 2

The polymerizable silicone monomer of the present invention can also be obtained by subjecting an organosilicon compound represented by the following formula (4) to a hydrolytic condensation reaction with a hydrolyzable organosilicon compound represented by the following formula (5) and/or a disiloxane compound represented by the following formula (6) in the presence of an acid. 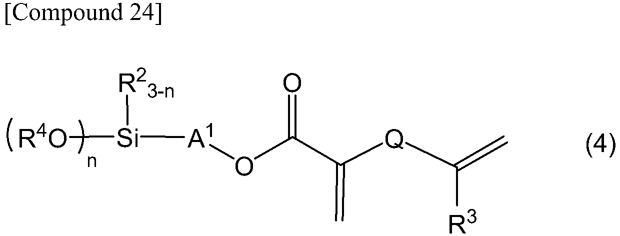 [Compound 25]

Y-SiR¹₃ (5)

**R¹₃SiOSiR¹₃** (6)

R¹, R², R³, A¹, Q and n are as defined above.

In formula (4), R⁴ is, independently of each other, an alkyl group having 1 to 4 carbon atoms. Examples of R⁴ include methyl, ethyl, n-propyl, isopropyl, n-butyl, isobutyl, and *tert-butyl* groups. Among them, a methyl group and an ethyl group are more preferred.

Y is a chlorine atom, a bromine atom, or an iodine atom.

More specifically, the carboxylic acid metal salt compound represented by formula (4) is represented by the following structure.

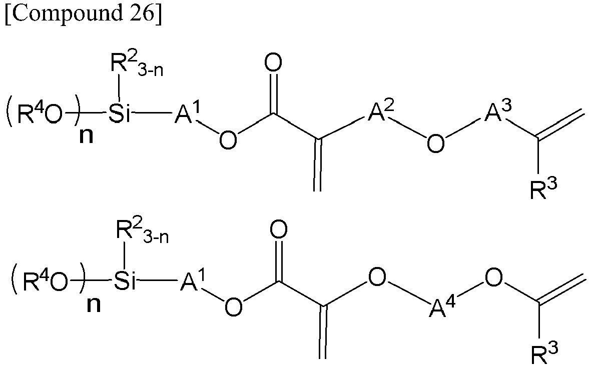

A², A³, and A⁴ are as defined above, and are preferably groups selected from the following structures. In another preferred embodiment, either A² or A³ is a single bond, and the other of A² and A³ is a group selected from the following formula.

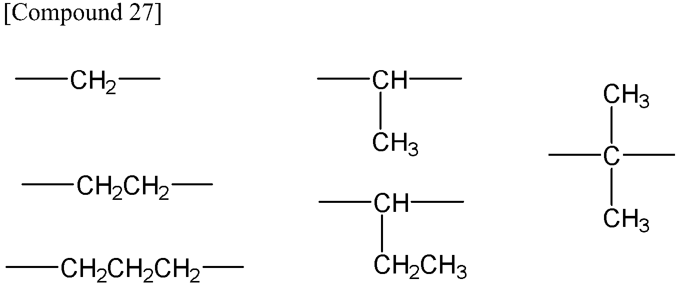

Most preferably, A², A³, and A⁴ are a methylene group.

Examples of the organosilicon compound represented by formula (4) include those represented by the following formulas (12) to (17): 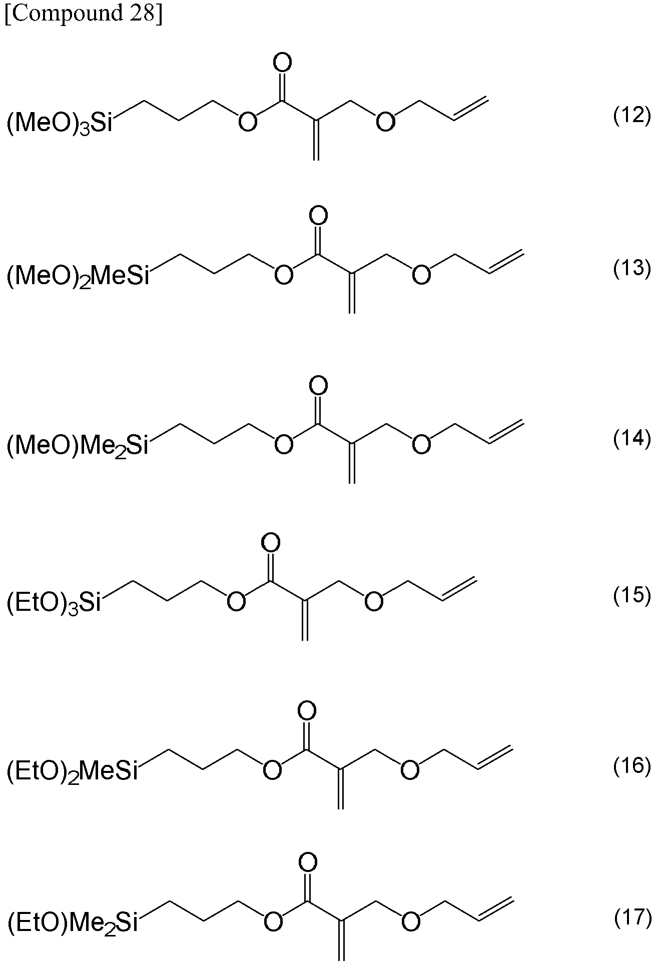 wherein Me represents a methyl group, and Et represents an ethyl group.

Examples of the hydrolyzable organosilicon compound represented by formula (5) include trimethylchlorosilane, triethylchlorosilane, trimethylbromosilane, triethylbromosilane, trimethyliodosilane, and triethyliodosilane.

Examples of the disiloxane compound represented by formula (6) include hexamethyldisiloxane and hexaethyldisiloxane.

It is preferred that the organosilicon compound represented by formula (4) be reacted with the hydrolyzable organosilicon compound represented by formula (5) at a molar ratio of OR⁴ groups in the organosilicon compound to the hydrolyzable organosilicon compound of 1:1 to 1:10, particularly from 1:2 to 1:5.

It is preferred that the organosilicon compound represented by formula (4) be reacted with the disiloxane compound represented by formula (6) at a molar ratio of OR⁴ groups in the organosilicon compound to R¹₃Si groups in the disiloxane compound of 1:1 to 1:10, particularly from 1:2 to 1:5.

The hydrolyzable organosilicon compound represented by formula (5) and the disiloxane compound represented by formula (6) may be mixed and used in amounts such that the aforementioned ratios with respect to the organosilicon compound represented by formula (4) are satisfied.

Examples of the acid include hydrochloric acid, sulfuric acid, concentrated sulfuric acid solution, methanesulfonic acid, trifluoromethanesulfonic acid, acetic acid, acetic anhydride, and trifluoroacetic acid; and the acid is particularly preferably hydrochloric acid and sulfuric acid. The amount of the acid used is preferably 0.1 to 50 mol, particularly preferably 1 to 10 mol, per mol of the organosilicon compound represented by formula (4).

A solvent may be used in the hydrolytic condensation reaction, if necessary. Examples of the solvent include alcoholic solvents such as methanol, ethanol, 1-propanol, and 2-propanol. These solvents may be used alone or in combination of two or more thereof.

For example, the hydrolytic condensation reaction may be carried out by a method in which the organosilicon compound represented by formula (4) and the hydrolyzable organosilicon compound represented by formula (5) are added to a mixture of an aqueous hydrochloric acid solution and a solvent, or by a method in which sulfuric acid, the organosilicon compound represented by formula (4), and water are sequentially added to a mixture of the disiloxane compound represented by formula (6) and a solvent.

The reaction temperature of the hydrolytic condensation reaction is preferably - 10 to 50°C, particularly preferably -5 to 30°C. Furthermore, the reaction time is not particularly limited, and typically is about 1 to 30 hours, preferably 1 to 20 hours, more preferably 1 to 10 hours.

The acid used as described above can be removed by phase separation to remove the layer containing the acid, which is preferred.

The reaction mixture obtained after the hydrolytic condensation reaction is washed with water, an aqueous sodium bicarbonate solution, an aqueous sodium sulfate solution, or the like, then dehydrated with anhydrous sodium sulfate, calcium chloride, or the like, followed by purification by distillation under reduced pressure to obtain the polymerizable silicone monomer of the present invention.

When preparing the polymerizable silicone monomer of the present invention, a polymerization inhibitor is preferably added, and it is more desirable that the polymerization inhibitor be present together with a molecular oxygen-containing gas. Typically, air or oxygen gas diluted with nitrogen or another inert gas is used as the molecular oxygen-containing gas and is introduced into the handling equipment.

A polymerization inhibitor for a radical-polymerizable monomer can be used as the polymerization inhibitor. Examples of the polymerization inhibitor include phenol-based inhibitors such as hydroquinone, methylhydroquinone, trimethylhydroquinone, t-butylhydroquinone, *p*-methoxyphenol, 2,6-di-*t*-butyl-*p*-cresol, 6-*t*-butyl-2,4-xylenol, 2,6-di-*t*-butylphenol, 2,6-di-*t*-butyl-4-methoxyphenol, 2,2'-methylenebis(4-methyl-6-*t-*butylphenol); organic acid copper salts; and phenothiazine. Among them, phenol-based inhibitors are preferred in view of low coloration and polymerization inhibition, among which *p*-methoxyphenol, 2,6-di-*t*-butyl-p-cresol, 6-*t*-butyl-2,4-xylenol, and 2,6-di-*t-*butylphenol are preferred in view of availability and economy. These polymerization inhibitors may be used alone or in combination of two or more thereof. The amount of the polymerization inhibitor added is not particularly limited, but is typically about 100 to 100,000 ppm based on the mass of the metal salt compound represented by formula (3) or the organosilicon compound represented by formula (4).

According to the preparation method of the present invention, a compound is provided that contains, at high purity, a single type of compound represented by formula (1) having one specific n, A¹, Q, R¹, R² and R³. Herein, "high purity" means that the single type of compound having the specific structure accounts for more than 95% by mass, preferably 97% by mass or more, and more preferably 99% by mass or more, based on the total mass of the compound. In the present invention, the purity is determined by gas chromatography (hereinafter referred to as "GC") analysis. Detailed conditions will be described below. The high purity of the compound as described above makes it possible to obtain a more transparent polymer.

### Curable composition

The curable composition of the present invention contains the polymerizable silicone monomer represented by formula (1). The content of the polymerizable silicone monomer is not particularly limited, and is preferably 10 to 80% by mass, more preferably 20 to 70% by mass of the composition. When a solvent is incorporated in the composition, "the content" refers to the nonvolatile component excluding the solvent.

The curable composition of the present invention preferably contains another compound (hereinafter, referred to as a polymerizable monomer) having a group that polymerizes with the polymerizable silicone monomer represented by formula (1). Examples of the polymerizable monomer include acrylic monomers such as (meth)acrylic acid, methyl(meth)acrylate, ethyl(meth)acrylate, (poly)ethylene glycol dimethacrylate, polyalkylene glycol mono (meth)acrylate, polyalkylene glycol monoalkylether (meth)acrylate, trifluoroethyl(meth)acrylate, triethylene glycol dimethacrylate, 2-hydroxyethyl(meth)acrylate, and 2,3-dihydroxypropyl(meth)acrylate; acrylic acid derivatives such as *N,N*-dimethylacrylamide, *N,N-*diethylacrylamide, *N-*acryloylmorpholine, and *N*-methyl(meth)acrylamide; other unsaturated aliphatics or aromatic compounds such as crotonic acid, cinnamic acid, and vinyl benzoate; and silicone monomers having a (meth)acrylic group or other polymerizable group. These may be used alone or in combination of two or more types thereof.

### Polymer

Copolymerization of the polymerizable silicone monomer of the present invention and the aforementioned other polymerizable monomer may be carried out by a conventionally-known method. For example, the copolymerization can be carried out using a known polymerization initiator such as a thermal polymerization initiator or a photopolymerization initiator. Examples of the polymerization initiator include 2-hydroxy-2-methyl-1-phenyl-propan-1-one, azobisisobutyronitrile, azobisdimethylvaleronitrile, benzoyl peroxide, *tert*-butyl hydroperoxide, and cumene hydroperoxide. These polymerization initiators can be used alone or in combination of two or more types thereof. The amount of the polymerization initiator is preferably 0.001 to 2 parts by mass, more preferably 0.01 to 1 part by mass, relative to total 100 parts by mass of the polymerization component. The polymerization reaction may be carried out under conventionally-known reaction conditions. For example, methods include a method of photopolymerization by, for example, irradiating with light containing ultraviolet light from a mercury lamp or an ultraviolet lamp for 1 hour or less, or a method of thermal polymerization for, for example, 0.1 hours to several hours at a temperature of, for example, 60°C to 200°C.

### Ophthalmic device

The present invention preferably provides an ophthalmic device comprising a copolymer of a monomer mixture containing the polymerizable silicone monomer of the present invention and the aforementioned other polymerizable monomer. The content ratio of the polymerizable silicone monomer of the present invention in the monomer mixture is preferably 10 to 80 parts by mass, more preferably 20 to 70 parts by mass, relative to 100 parts by mass of the monomer mixture. If necessary, the monomer mixture may further contain a known additive such as an antioxidant, a colorant, a lubricant, an internal wetting agent, and a reinforcing agent as long as objects and effects of the present invention are not impaired.

A polymer containing the polymerizable silicone monomer of the present invention as a polymerization component exhibits excellent transparency, surface hydrophilicity, durability, and stain resistance. Accordingly, the polymer is suitable for use in producing ophthalmic devices, such as contact lenses, intraocular lenses, and artificial corneas. The method for producing an ophthalmic device using the polymer is not particularly limited, and any conventionally known method for producing ophthalmic devices may be employed. For example, when forming the polymer into the shape of a lens, such as a contact lens or an intraocular lens, cutting or casting (molding) methods may be used.

### EXAMPLES

The present invention will be explained more specifically with reference to the following Examples and Comparative Examples; however, the present invention is not limited to the following Examples.

In the following Examples, ¹H NMR analysis was carried out using a JNM-ECP500 (manufactured by JEOL Ltd.), with deuterated chloroform used as the measurement solvent.

Furthermore, in the following description, the purities of the compounds were determined by gas chromatography (GC) measurement under the following conditions.

### Gas chromatography (GC) measurement conditions:

Gas chromatograph: manufactured by Agilent Technologies Japan, Ltd.
Detector: FID, temperature: 300°C
Capillary column: J&W HP-5MS (0.25 mm×30m×0.25 µm)
Temperature program: 50°C (5 minutes) → 10°C/min → 250°C (maintain)
Inlet temperature: 250°C
Carrier gas: helium (1.0 ml/min)
Split ratio: 50:1
Injection volume: 1 µl

### Example 1

### Silicone Compound 1 synthesis (Production Method 1)

In a 1-L separable flask equipped with a stirrer, a reflux condenser, a dropping funnel, and a thermometer were placed 150.0 g (0.833 mol) of 2-(allyloxymethyl) potassium acrylate, 75 g of toluene, 0.56 g of 2,6-di-tert-butyl-p-cresol, and 5.2 g of tetrabutylammonium bromide. To this mixture was added 295.6 g (0.793 mol) of 3-chloropropyl tris(trimethylsiloxy)silane dropwise over 1 hour while maintaining the internal temperature at 95 to 105 °C. Thereafter, the reaction mixture was stirred at 100 °C for 3 hours, and gas chromatographic analysis confirmed the disappearance of 3-chloropropyl tris(trimethylsiloxy)silane. The resulting solution was purified by distillation under conditions of 7 mmHg and 150 °C to obtain 183.6 g of a colorless and transparent liquid (Silicone Compound 1) with a purity of 98.7%.

¹H-NMR measurements confirmed that the thus-obtained Silicone Compound 1 had the structure represented by the following formula (9).

¹H-NMR (CDCl₃, 500 MHz) δ: 0.1 (s, 27H), 0.5 (t, 2H), 2.7 (m, 2H), 4.05 (d, 2H), 4.15 (t, 2H), 4.2 (s, 2H), 5.15-5.25 (m, 2H), 5.7 (s, 1H), 5.7-6.0 (m, 1H), 6.3 (s, 1H).

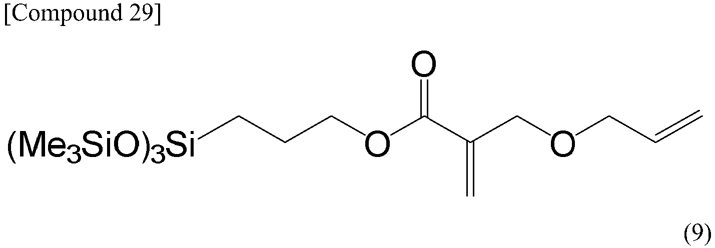

### Example 2

### Silicone Compound 1 synthesis (Production Method 2)

In a 2-L separable flask equipped with a stirrer, a reflux condenser, a dropping funnel, and a thermometer were placed 115.1 g of a 35% aqueous hydrochloric acid solution, 115.1 g of water, and 230.3 g of 2-propanol. To this mixture were added dropwise 171.2 g (0.563 mol) of an organosilicon compound represented by the following formula (12) and 550.0 g (5.069 mol) of trimethylchlorosilane over 1 hour while maintaining the internal temperature at -3 to 15 °C. Thereafter, the reaction mixture was stirred at 25 °C for 2 hours. Subsequently, 115.1 g of water was added to the reaction mixture, and the mixture was allowed to stand. The lower aqueous hydrochloric acid layer was then removed by phase separation, and washing with water was repeated five times. To the thus-obtained upper layer solution, was added 0.23 g of 2,6-di-tert-butyl-p-cresol, followed by purification by distillation under conditions of 7 mmHg and 150 °C to obtain 120.0 g of a colorless and transparent liquid (Silicone Compound 1) with a purity of 98.4%.

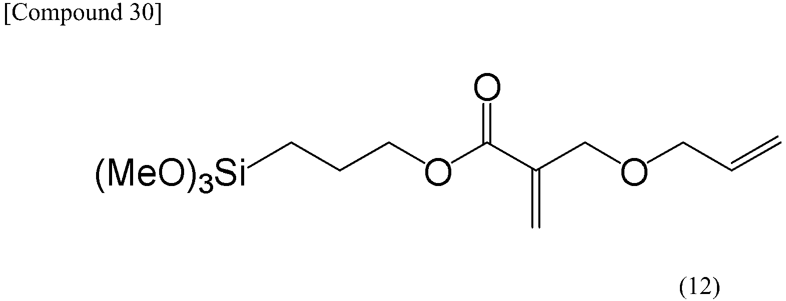

¹H-NMR measurements confirmed that the thus-obtained Silicone Compound 1 had the structure represented by said formula (9).

¹H-NMR (CDCl₃, 500 MHz) δ: 0.1 (s, 27H), 0.5 (t, 2H), 2.7 (m, 2H), 4.05 (d, 2H), 4.15 (t, 2H), 4.2 (s, 2H), 5.15-5.25 (m, 2H), 5.7 (s, 1H), 5.7-6.0 (m, 1H), 6.3 (s, 1H).

### Example 3

### Silicone Compound 2 synthesis

The procedure of Example 1 was repeated, except that 250.0 g (0.793 mol) of 3-chloropropylmethyl bis(trimethylsiloxy)silane was used in place of the 3-chloropropyl tris(trimethylsiloxy)silane of Example 1, to thereby obtain 155.3 g of a colorless and transparent liquid (Silicone Compound 2) with a purity of 98.5%.

¹H-NMR measurements confirmed that the thus-obtained Silicone Compound Silicone Compound 2 had the structure represented by the following formula (10).

¹H-NMR (CDCl₃, 500 MHz) δ: 0.1 (s, 21H), 0.5 (t, 2H), 2.7 (m, 2H), 4.05 (d, 2H), 4.15 (t, 2H), 4.2 (s, 2H), 5.15-5.25 (m, 2H), 5.7 (s, 1H), 5.7-6.0 (m, 1H), 6.3 (s, 1H).

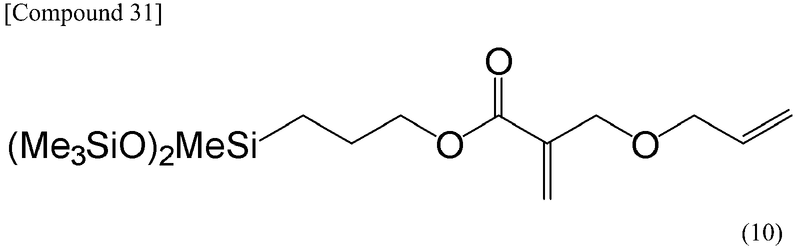

### Monomer mixture preparation

### Example 4

Silicone Compound 1 obtained in Example 1 (60 parts by mass), N,N-dimethylacrylamide (35 parts by mass), triethylene glycol dimethacrylate (1 part by mass), trifluoroethyl (meth)acrylate (5 parts by mass), and Darocur 1173 (manufactured by Ciba Specialty Chemicals plc; 0.5 parts by mass) were mixed and stirred to obtain Monomer Mixture 1.

### Example 5

In Example 5, Monomer Mixture 2 was prepared using the same composition and method as in Example 4, except that Silicone Compound 1 was replaced with Silicone Compound 2.

### Comparative Example 1

In Comparative Example 1, Monomer Mixture 3 was prepared using the same composition and method as in Example 4, except that Silicone Compound 1 was replaced with Silicone Compound 3 (3-[tris(trimethylsiloxy)silyl]propyl methacrylate, manufactured by Gelest Inc.).

### Evaluation test

### (1) Compatibility with Other Polymerizable Monomer

The appearances of Monomer Mixtures 1 to 3 obtained above were visually observed. Mixtures in which the compatibility between the silicone compound and the other (meth)acrylic compound was good were colorless and transparent, whereas mixtures in which the compatibility was poor became cloudy. The results are shown in Table 1 below.

### (2) Film (polymer) Appearance

Each of Monomer Mixtures 1 to 3 obtained above was degassed under an argon atmosphere. The mixture was poured into a mold sandwiched between two quartz glass plates, and irradiated with an ultra-high-pressure mercury lamp for 1 hour to obtain a film having a thickness of about 0.3 mm. The appearance of the film was visually observed. The results are shown in Table 1 below.

### (3) Film (Polymer) Surface Hydrophilicity

The water contact angle (°) of the film produced in item (2) was measured by the sessile drop method using a contact angle meter, CA-D type (manufactured by Kyowa Interface Science Co., Ltd.). The results are shown in Table 1 below.

### (4) Film (Polymer) Stain Resistance

The film produced in item (2) was immersed in a phosphate-buffered saline solution (PBS (-)) at 37 °C for 24 hours. Each film before immersion in PBS (-) and each film after immersion for 24 hours were incubated in a known artificial lipid solution at 37 °C ± 2 °C for 8 hours. Thereafter, each film was rinsed with PBS (-) and immersed in a 0.1% Sudan black-sesame oil solution. Films for which no difference in staining before and after immersion was observed were classified as "G", whereas films for which staining from colorless to black was observed were classified as "NG". The results are shown in Table 1 below.

### (5) Film (Polymer) Durability

Two films were produced in accordance with item (2). The surface moisture of one of the films was wiped off, and the film was then immersed in a phosphate-buffered saline solution (PBS (-)) at 37 °C for 24 hours. Each film before immersion in PBS (-) and each film after immersion for 24 hours was cut into a dumbbell shape having a width of 2.0 mm. The upper and lower ends of each test sample were clamped with jigs, and the breaking strength and the elongation at break upon continuous pulling at a constant rate were measured using a tensile testing machine, AGS-50NJ (manufactured by Shimadzu Corporation). When the changes in both the breaking strength and the elongation at break before and after immersion in PBS (-) were within 10%, the sample was evaluated as "G". When a decrease of more than 10% was observed in either the breaking strength or the elongation at break, the sample was evaluated as "NG". The results are shown in Table 1 below.

**[Table 1]**

| | Example 4 | Example 5 | Comparative Example1 |
|---|---|---|---|
| Monomer Mixture | 1 | 2 | 3 |
| (1) Compatibility with Monomer | Colorless and transparent | Colorless and transparent | Slightly cloudy |
| (2) Film Appearance | Colorless and transparent | Colorless and transparent | Slightly cloudy |
| (3) Surface Hydrophilicity (°) | 45 | 42 | 54 |
| (4) Stain Resistance | G | G | NG |
| (5) Durability | G | G | NG |

As shown in Comparative Example 1, Silicone Compound 3 had poor compatibility with other (meth)acrylic monomer compounds and did not provide a transparent polymer. In addition, the polymer thus obtained exhibited poor surface hydrophilicity and stain resistance, and its mechanical strength was reduced upon immersion in a phosphate-buffered saline solution. In contrast, the polymerizable silicone monomer of the present invention exhibited good compatibility with other (meth)acrylic monomers, was colorless and transparent, and provided a polymer having excellent surface hydrophilicity and stain resistance. Furthermore, the polymer did not exhibit a reduction in mechanical strength even when immersed in a phosphate-buffered saline solution.

### INDUSTRIAL APPLICABILITY

The polymerizable silicone monomer of the present invention provides a polymer that is colorless and transparent and exhibits excellent surface hydrophilicity and stain resistance. Furthermore, a polymer containing repeating units derived from the polymerizable silicone monomer of the present invention does not exhibit a reduction in mechanical strength even when immersed in a phosphate-buffered saline solution, and therefore has excellent durability. In addition, the preparation method of the present invention makes it possible to provide a compound having a specific structure as a single type of compound with high purity. Accordingly, the polymerizable silicone monomer of the present invention and the method for preparing the polymerizable silicone monomer are useful for producing ophthalmic devices, such as contact lens materials, intraocular lens materials, and artificial cornea materials.

## Claims

1. A polymerizable silicone monomer represented by the following formula (1): 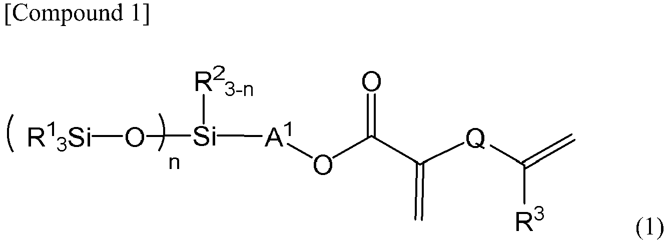
wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms,
R³ is a hydrogen atom or a methyl group,
A¹ is a single bond or a divalent hydrocarbon group having 1 to 20 carbon atoms,
Q is a divalent group selected from the group consisting of -A²-O-A³- and -O-A⁴-O-, A² and A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, provided that A² and A³ are not both single bonds,
A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and n is an integer of 1 to 3.

2. The polymerizable silicone monomer according to claim 1, represented by the following formula (a) or (b): 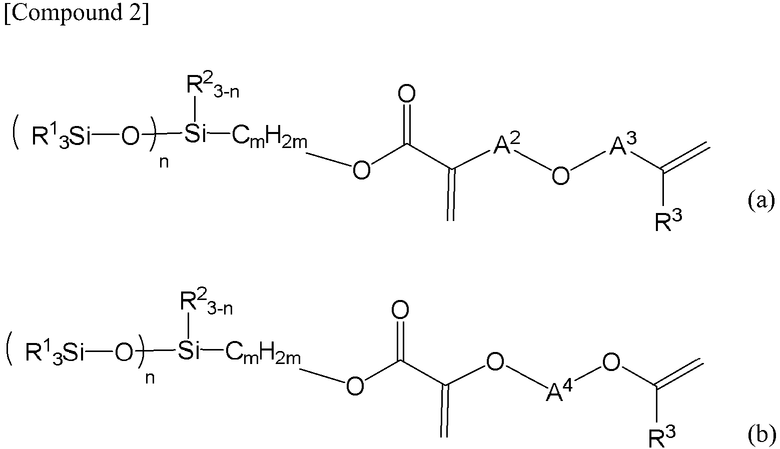 wherein R¹, R², R³, A², A³, A⁴, and n are as defined above, and m is an integer of 1 to 10.

3. The polymerizable silicone monomer according to claim 1, wherein A², A³, and A ⁴ are a group selected from the following groups.

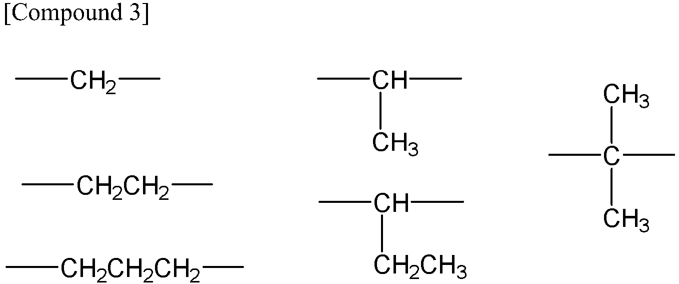

4. The polymerizable silicone monomer according to claim 1, wherein either A² or A³ is a single bond, and the other of A² and A³ is a group selected from the following groups.

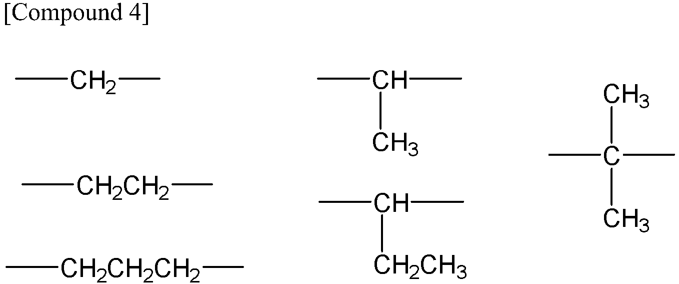

5. The polymerizable silicone monomer according to claim 1, wherein A², A³, and A⁴ are a methylene group.

6. A method for preparing a polymerizable silicone monomer represented by the following formula (1): 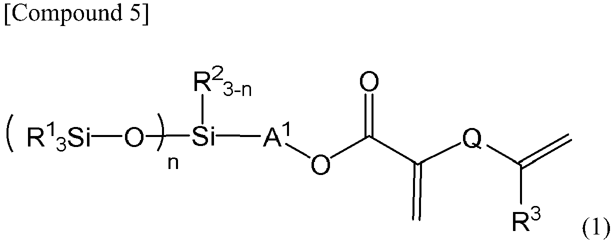
wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms,
R³ is a hydrogen atom or a methyl group,
A¹ is a single bond or a divalent hydrocarbon group having 1 to 20 carbon atoms,
Q is a divalent group selected from the group consisting of -A²-O-A³- and -O-A⁴-O-, A² and A³ are, independently of each other, a single bond or an optionally branched divalent hydrocarbon group having 1 to 3 carbon atoms, wherein at least one hydrogen atom bonded to a carbon atom of the divalent hydrocarbon group may be substituted with an alkoxy group having 1 to 4 carbon atoms, provided that A² and A³ are not both single bonds,
A⁴ is an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, and n is an integer of 1 to 3,
wherein the method comprises a step of reacting a halogen group-containing organosilicon compound represented by the following formula (2): 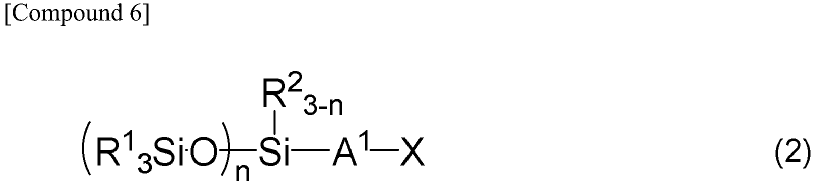
with a carboxylic acid metal salt compound represented by the following formula (3): 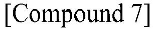 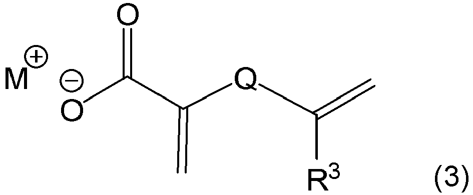
wherein R¹, R², R³, A¹, Q, and n are as defined above, X is a chlorine atom, a bromine atom, or an iodine atom, and M is an alkali metal ion,
to obtain the polymerizable silicone monomer represented by said formula (1).

7. The method according to claim 6, wherein A², A³, and A⁴ are groups selected from the following groups.

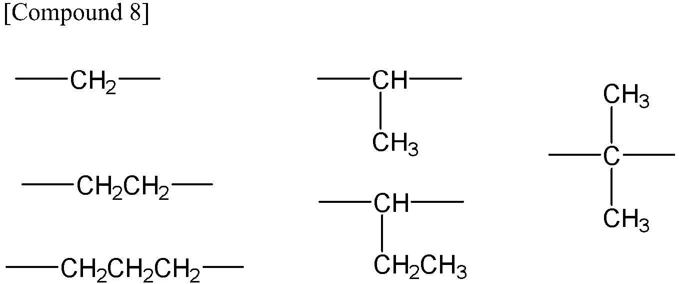

8. The method according to claim 6, wherein either A² or A³ is a single bond, and the other of A² and A³ is a group selected from the following groups.

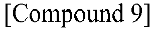

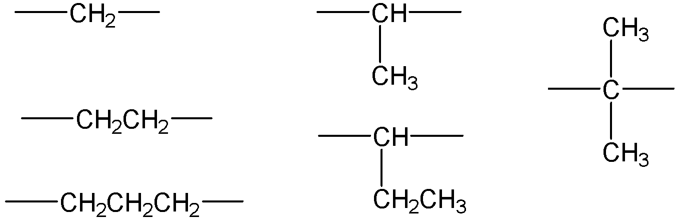

9. The method according to claim 6, wherein A², A³, and A⁴ are a methylene group.

10. A method for preparing a polymerizable silicone monomer represented by the following formula (1): 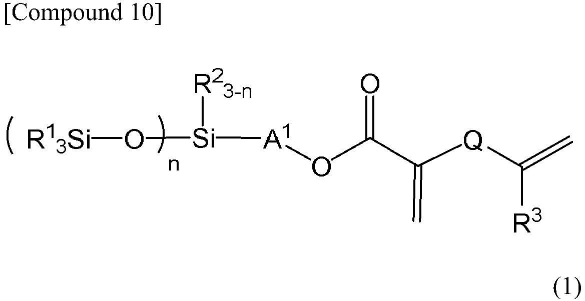
wherein R¹ is, independently of each other, a monovalent hydrocarbon group having 1 to 4 carbon atoms, R² is, independently of each other, an alkyl group having 1 to 10 carbon atoms or an aryl group having 6 to 10 carbon atoms,
R³ is a hydrogen atom or a methyl group,
A¹ is a single bond or a divalent hydrocarbon group having 1 to 20 carbon atoms,
Q is a divalent group selected from the group consisting of -A²-O-A³- and -O-A⁴-O-, A² and
A³ are, independently of each other, a single bond or an optionally branched divalent hydrocarbon group having 1 to 3 carbon atoms wherein at least one hydrogen atom bonded to a carbon atom of the divalent hydrocarbon group may be substituted with an alkoxy group having 1 to 4 carbon atoms, provided that A² and A³ are not both single bonds,
A⁴ is an optionally branched divalent hydrocarbon group having 1 to 3 carbon atoms
wherein at least one hydrogen atom bonded to a carbon atom of the divalent hydrocarbon group may be substituted with an alkoxy group having 1 to 4 carbon atoms, and n is an integer of 1 to 3,
wherein the method comprises a step of subjecting an organosilicon compound represented by the following formula (4): 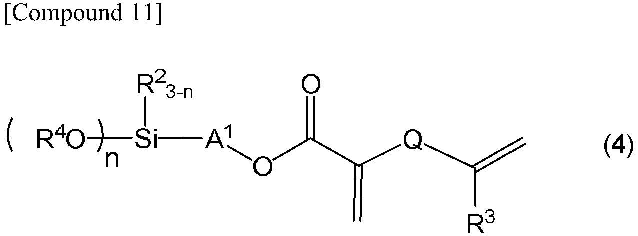
to a hydrolytic condensation reaction in the presence of an acid with a hydrolyzable organosilicon compound represented by the following formula (5) and/or a disiloxane compound represented by the following formula (6):
[Compound 12]
V-SiR¹₃ (5)
**R¹₃SiOSiR¹₃** (6)
wherein R¹, R², R³, A¹, Q, and n are as defined above, R⁴ is an alkyl group having 1 to 4 carbon atoms, and Y is a chlorine atom, a bromine atom, or an iodine atom,
to obtain the polymerizable silicone monomer represented by said formula (1).

11. The method according to claim 10, wherein A², A³, and A⁴ are a group selected the following groups.

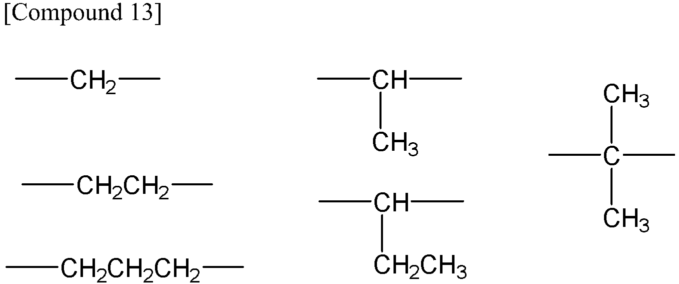

12. The method according to claim 10, wherein either A² or A³ is a single bond, and the other of A² and A³ is a group selected from the following groups.

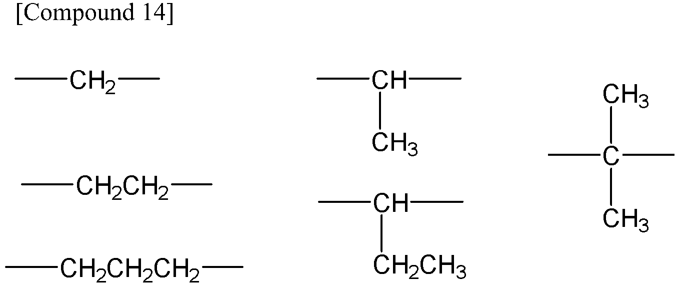

13. The method according to claim 10, wherein A², A³, and A⁴ are a methylene group.

14. A curable composition comprising the polymerizable silicone monomer according to any one of claims 1 to 5 and another polymerizable monomer having a group that polymerizes with the polymerizable silicone monomer.

15. The curable composition according to claim 14, comprising 10 to 80% by mass of the polymerizable silicone monomer according to any one of claims 1 to 5, based on the curable composition.

16. A polymer obtained by polymerizing the curable composition according to claim 15.

17. An ophthalmic device comprising the polymer according to claim 16.
